Europäisches Patentamt
European Patent Office
Office européen des brevets

(11) Publication number: 0 345 662 A1

# EUROPEAN PATENT APPLICATION

(21) Application number: 89110021.6

(22) Date of filing: 02.06.89

(51) Int. Cl.4: C08L 27/06 , C08K 5/00 , A61L 2/08 , //(C08K5/00,5:11, 5:13)

(30) Priority: 06.06.88 US 202607

(43) Date of publication of application: 13.12.89 Bulletin 89/50

(84) Designated Contracting States: AT BE CH DE ES FR GB GR IT LI LU NL SE

(71) Applicant: THE B.F. GOODRICH COMPANY
3925 Embassy Parkway
Akron Ohio 44313-1799(US)

(72) Inventor: Forsyth, Thomas Henry
1924 26th Street
Cuyahoga Falls Ohio 44223(US)
Inventor: Summers, James William
29751 Wolf Road
Bay Village Ohio 44140(US)
Inventor: Toyoda, Paul Scott
5342 Barkwood Drive
Sheffield Village Ohio 44054(US)
Inventor: Kline, Sally Ann
90 Sussex Road
Hudson Ohio 44236(US)
Inventor: Rabinovitch, Elvira Borisovna
3798 Colony Road
South Euclid Ohio 44118(US)

(74) Representative: von Kreisler, Alek, Dipl.-Chem. et al
Deichmannhaus am Hauptbahnhof
D-5000 Köln 1(DE)

## (54) Rigid vinyl polymer articles resistant to discoloration from gamma radiation.

(57) The present invention resides in a clear, rigid, vinyl polymer part sterilized by exposure to about 2 to about 5 Mrad of gamma radiation characterized as having an increase in yellowness index from radiation, as determined by ASTM D1925-70, of less than about 10, and a rigidity as determined by ASTM Tensile Test D638 of more than about 250,000 psi.

EP 0 345 662 A1

# RIGID VINYL POLYMER ARTICLES RESISTANT TO DISCOLORATION FROM GAMMA RADIATION

## Background of the Invention

The present invention relates to vinyl polymer compounds which retain their optical properties and toughness when exposed to high energy ionizing radiation. The present invention will be described with respect to clear, hard, rigid plastic parts, such as rigid Y's for flexible tubing employed in the medical field, although it will be apparent to those skilled in the art that the present invention has other applications, such as connectors for medical IV sets, blood filters, clamps, packaging and containers.

## Brief Description of the Prior Art

It is well known to package components or assemblies of components used in the medical field in sterile packages for storage until use. Frequently included in such packages are rigid components such as Y tubing connectors.

For some time, a preferred method for sterilization of the packaged components and assemblies has been by cobalt 60 gamma radiation. However, gamma sterilization is known to have its drawbacks. For example, many polymers, particularly ordinary rigid vinyl polymer compositions, show severe discoloration and degradation following radiation exposure.

This is discussed in an article entitled "Gamma Sterilization of Rigid and Flexible PVC", published in Society of Plastics Engineering, Technical Papers, Vol. XXXIV, 1988, pages 1344-1347, E. Mertzel, J. Summers, and T. Forsyth; and also in a publication entitled "The Stabilization of PVC Against Gamma Radiation", Medical Device and Diagnostic Industry, November 1983, pages 57-61, Foure and Rakita.

In the Mertzel et al. publication, it is indicated that one of the major mechanisms of degradation is chain scission. "Chain scission occurs as a random breaking of bonds which reduces the molecular weight of the resin. The breaking of bonds in vinyl resins can also cause the evolution of HCl and the occurrence of unsaturation in the vinyl backbone. This unsaturation results in the yellowing and/or the loss of clarity in the product."

Another mechanism involved in the polymer degradation is crosslinking. It is stated in the Mertzel et al. publication: "Crosslinking, produced by the combination of two polymer radicals results in the formation of 3-dimensional networks. With crosslinking, there are changes in mechanical properties, such as tensile strength, elastic modulus, impact strength, shear strength, brittleness, and elongation."

The disclosure of the Mertzel et al. publication is incorporated by reference herein.

In the December issue of Medical Device and Diagnostic Industry, 1983, on pages 33-37, the authors Foure and Rakita disclose a class of compounds marketed by M & T Chemicals, Rahway, New Jersey, under the trademark "Gammashield", which are said to prevent the discoloration and loss of physical properties. These compounds are described as organotin, organoantimony, and the calcium/zinc versions thereof. The intended uses disclosed for the compounds are rigid, hard polyvinyl chloride bottles or what is referred to as "blister packaging".

A similar disclosure is contained in prior U.S. Patent No. 4,412,897, isssued on November 1, 1983 to Kornbaum et al. One commercial additive marketed for gamma radiation resistance is Gammashield 801, a composition believed to contain organotin, a benzoate or terphthalate ester and an alkyl phenol. Rigid compositions containing this compound have unacceptably poor color retention on exposure to gamma radiation.

A prior publication entitled "Factors Influencing Color Drift of Gamma Sterilized PVC Articles", by David S.

Housel, Society of Plastics Engineering, Technical Papers, Vol. XXXI, 1985, pages 1068-1071, also discusses the problem of gamma sterilization of polyvinyl chloride and severe discoloration. Housel in this publication discloses that in comparative tests comparing dioctyladipate and two phthalate-type plasticizers, at levels of 50, 60 and 70 phr (parts per hundred parts of resin), dioctyladipate was found to be more efficient in retarding color drift after exposure to gamma radiation.

In prior patent No. 4,640,819, reference is made to the use of extruded polyvinyl tubing and gamma irradiation for sterilization. However, the tubing was flexible tubing plasticized with a suitable plasticizer. The problem of discoloration is not normally experienced with plasticized tubing, and appears to be limited to

rigid parts which are substantially non-plasticized. In the '819 patent, the rigid parts are preferably made from a polycarbonate.

## Summary of the Invention

Broadly, the present invention resides in a clear, rigid, vinyl polymer part sterilized by exposure to about 2 to about 5 Mrad of gamma radiation characterized as having an increase in yellowness index from radiation, as determined by ASTM D1925-70, of less than about 10, and a rigidity as determined by ASTM Tensile Test D638 of more than about 250,000 psi.

The present invention also resides in the discovery that the stabilization of vinyl polymer parts against gamma radiation is unexpectedly enhanced by the addition to the polymer of about 2 to about 20 phr of an ether-containing adipate or glutarate plasticizer in combination with about 0.2 to about 5 phr of butylated hydroxy toluene.

In a preferred embodiment of the present invention, the vinyl polymer composition also comprises about 0.2 phr to about 5 phr of a dialkyl tin stabilizer, more preferably about 2-3 phr, wherein the alkyl group in said stabilizer has 1-8 carbon atoms.

An advantage of the present invention is that parts prepared in accordance with the invention can be further characterized as having good heat stability.

## Detailed Description of the Present Invention and Best Mode

Broadly, the vinyl polymer of the present invention can be any homopolymer or copolymer of vinyl halides or vinylidene halides such as vinyl chloride, vinyl bromide, and vinylidene chloride. The vinyl halides and vinylidene halides may be copolymerized with each other or each with one or more polymerizable olefinic monomers having at least one terminal $CH_2=C<$ grouping. As examples of such olefinic monomers, there may be mentioned the alpha, beta-olefinically unsaturated carboxylic acids, such as acrylic acid, methacrylic acid, ethacrylic acid, alpha-cyanoacrylic acid, and the like; esters of acrylic acid, such as methyl acrylate, ethyl acrylate, butyl acrylate, octy acrylate, cyanoethyl acrylate, and the like; esters of methacrylic acid, such as methyl methacrylate, butyl methacrylate, and the like, nitriles, such as acrylonitrile, methacrylonitrile, and the like; acrylamides, such as methyl acrylamide, N-methylol acrylamide, N-butoxy methyacrylamide, and the like; vinyl ethers, such as ethyl vinyl ether, chlorethyl vinyl ether, and the like; the vinyl ketones; styrene and styrene derivatives, such as methyl styrene, vinyl toluene, chlorostyrene, and the like; vinyl naphthalene, allyl and vinyl chloroacetate, vinyl acetate, vinyl pyridine, methyl vinyl ketone; the diolefins, including butadiene, isoprene, chloroprene, and the like; and other polymerizable olefinic monomers of the types known to those skilled in the art.

The present invention is particularly applicable to homopolymers and copolymers made by the polymerization of vinyl chloride or vinylidene chloride alone or in admixture with one or more polymerizable olefinic monomers copolymerizable therewith in amounts up to about 20% by weight, based on the weight of the monomer mixtures. Copolymers sometimes have a tendency to decrease the clarity of the article and therefore their amount should be minimized. The most preferred vinyl polymer, or resin, is polyvinyl chloride (PVC) homopolymer produced by mass polymerization or suspension polymerization, and the invention, for simplicity and convenience, will be described in connection therewith, it being understood that this is merely intended in an illustrative sense and not limitative.

### Plasticizer

A critical additive in the composition of the present invention is an adipate or glutarate plasticizer, preferably an ether-containing adipate or glutarate having a molecular weight less than about 2,000, more preferably less than about 500. Examples of suitable adipates are dioctyl adipate (DOA), dibutoxyethoxyethyl adipate (DBEEA), dibutoxyethyl adipate (DBEA), diisobutyl adipate (DIBA), diisooctyl adipate (DIOA). Examples of suitable glutarates are dibutoxyethoxyethyl glutarate (DBEEG) and dibutoxyethyl glutarate (DBEG). The function of these adipates and glutarates is to provide molecular mobility during and after radiation.

Suitable adipates and glutarates are marketed by C. P. Hall under the trademark "Plasthall" and also by Staflex Speciality Esters, Inc., Carteret, New Jersey, under the trademark "Staflex".

The amount of adipate or glutarate used is less than what is normally characterized as a plasticizing amount; that is, it is less than an amount required to obtain product flexibility. The amount which achieves product clarity without resulting in product flexibility is in the range of about 2 to about 20 phr.

Normally, in the composition of the present invention, the only plasticizer employed will be one of the above adipates or glutarates, or a combination thereof.

Stabilizer

A second critical ingredient of the composition of the present invention is a butylated hydroxy toluene (BHT) stabilizer present in an amount of about 0.2-5 phr. An ether-containing adipate or glutarate, alone, provides insufficient protection against gamma radiation. In combination with about 0.2 to about 5 phr of butylated hydroxy toluene, much improved color retention is achieved.

This improvement in color retention appears to be unique to a combination which includes butylated hydroxy toluene (BHT). Surprisingly, stabilizers chemically similar to BHT, such as butylated hydroxy anisole (BHA), hydroquinone, and propylgallate fail to offer the results achieved with BHT.

In the examples of this application, the butylated hydroxy toulene employed was 2;6-di-tert-butyl-4-methyl-phenol marketed by Borg-Warner Corporation under the trademark "Ultranox 226".

Even further improvement in color retention has been achieved by using a combination of stabilizers with the adipate or glutarate, in which one of the combination is butylated hydroxy toluene (BHT) and the other is an alkyl tin stabilizer having the formula

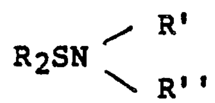

R in the above formula being a $C_1$ - $C_8$ group, $R'$ and $R''$ usually being the same organic group but different from R. Examples of such groups can contain oxygen or sulfur. Included in a listing of suitable groups are higher molecular weight polymerized radicals and octyl thioglycolate. Examples of suitable alkyl tin stabilizers are well known to those skilled in the art. In the present invention, any tin-based stabilizer which is FDA food approved, or has Class 6 approval, or even non-approval, can be used.

One alkyl tin stabilizer successfully employed is a dimethyl tin stabilizer wherein $R'$ and $R''$ are $-S-CH_2C-\overset{\overset{O}{\|}}{O}-C_8H_{17}$. This compound is marketed by Argus Chemical Co. under the trademark "MARK 1984". Another alkyl tin stabilizer successfully employed is a dioctyl tin stabilizer marketed by M & T Chemicals under the trademark "PA1753". Both stabilizers are FDA approved for food contact in PVC compounds.

One problem with tin stabilizers is their toxicity. In the present invention, they are employed at a sufficiently low level of about 0.2 to about 5 phr to be non-toxic. In tests conducted using the combination of a tin stabilizer with BHT, in combination with an ether-containing adipate or glutarate, articles exposed to up to 5 Mrads of gamma-ray or electron beam radiation showed only slight yellowing with no detrimental effect on material toughness.

Stabilizers added primarily to achieve heat stability can optionally be employed in the practice of the present invention. For instance, compositions have been successfully prepared employing an epoxy co-stabilizer, as it is commonly called in the polyvinyl chloride industry, to impart heat stability. Epoxidized soybean oil is frequently used for this purpose. Normally, the epoxidized soybean oils are added in the range of about 5 to about 20 phr, a conventional level of usage for heat stability without adversely affecting rigidity. Other stabilizers can also be used, if desired, with or in place of the epoxidized soybean oils, for instance, calcium/zinc, soap stabilizers. Such calcium/zinc stabilizers are fully disclosed in co-pending application Serial No. 768,987, filed August 26, 1985, by Roman W. Wypart, et al., assigned to assignee of the present application. The disclosure of co-pending application Serial No. 768,987 is incorporated by reference herein.

As disclosed in said application, at least one calcium soap and one zinc soap may be used at a level of from about 0.005 to about one phr, preferably from about 0.03 to about 0.3 phr. It is preferred that the level of zinc soap be greater than the level of calcium soap. The calcium soap level may be very low or even not

present at all. In the most preferred embodiment, the zinc soap is about twice the level of the calcium soap.

Examples of suitable calcium soaps are calcium stearate, calcium laurate, calcium oleate, calcium palmitate, calcium octoanate, and calcium benzoate. Calcium stearate is the preferred calcium soap. Examples of suitable zinc soaps are zinc stearate, zinc laurate, zinc palmitate, zinc 2-ethyl hexanate, zinc octoanate, zinc oleate, and zinc benzoate. Zinc stearate is a preferred zinc soap.

Other Additives

The compositions of the present invention can also use lubricants, impact modifiers, pigments, and processing aids following technology known to those skilled in the art.

Examples of suitable lubricants are polyglycerols of di- and trioleates. These esters are commercially available from a number of sources. Co-stabilizers that are well known in the art that can be employed are phosphite stabilizers such as polymeric phosphites, and beta-diketones.

Examples of suitable impact modifiers are acrylonitrile butadiene styrene terpolymers (ABS) and methacrylate butadiene styrene (MBS). These and others are disclosed in Plastics Compounding, Nov. Dec., 1983: "Update: Impact Modifiers for Rigid PVC", by Mary C. Mcmurrer, incorporated by reference herein.

Examples of processing aids which can be used are acrylic polymers such as disclosed in The Plastics and Rubber Institute; International Conference on PVC Processing, April 26-28 (1983), Paper No. 17. The processing aids function to improve the fluxing and melt properties of rigid PVC compounds. This paper is also incorporated by reference herein.

In the practice of the present invention, the ingredients of the composition are thoroughly mixed together to form the desired composition before it is shaped into the desired articles. Mixing may be accomplished by any of the customary methods known to those skilled in the art such as with a Banbury, Henschel, Mills, or other mixers.

The composition of the present invention may be formed into useful high clarity parts by different methods depending upon the desired end product. Standard techniques may be used to form the parts, such as by injection molding and extrusion molding. The wall thickness of parts made from the compositions of the present invention can vary from about 0.001 inches to about 0.15 inches, or thicker.

Color stability is determined by measuring the yellowness index according to ASTM D-1925 at different time intervals after subjecting samples to gamma radiation. In the standard test procedure, the samples are 0.065 inches in thickness. In some instances, tests were carried out on samples having a thickness of 0.040 inches. Recent samples prepared have a thickness of 0.125 inches. A number of colorimeters are commercially available designed to give direct readouts of yellowness index. One such instrument is manufactured by Gardner Laboratory, Inc. of Bethesda, Maryland, marketed under the trademark XL-10A. Another is one marketed under the trademark Hunterlab D25P by Hunter Associates Laboratory of Fairfax, Virginia.

The articles of the present invention preferably have a yellowness index change of less than about 10 when subjected to radiation up to about 5 Mrads.

Depending upon composition, for instance the presence of pigments, the parts may have an initial yellowness index reading. In such instance, the yellowness increase is the determinative factor rather than absolute yellowness index.

An important property of parts made in accordance with the present invention is rigidity. Rigidity is determined following the procedure of ASTM D638. Parts prepared following the teachings of the present invention have a rigidity (in terms of tensile modulus) of at least about 250,000 psi.

Another important property of the articles of the present invention is lack of or less toxicity. The major toxicological tests used on plastic parts are the tissue culture cytotoxicity test, and the USP VI test (USP intracutaneous test; and USP systemic injection test; and USP rabbit implantation test). Both of these tests are disclosed and discussed in the Mertzel publication, referred to above, and incorporated by reference herein. Toxicity tests conducted on the compositions of the present invention up to the present time suggest that they are at least less toxic than compositions of the prior art.

Physical properties such as tensile strength, elongation, and flexural strength, determined according to ASTM D637 and D790, showed no significant changes following exposure to gamma radiation.

Example 1

In this example, comparative data was obtained comparing the discoloration of different samples of polyvinyl chloride parts when exposed to gamma radiation. The degree of discoloration was determined subjectively by visual examination. The samples were basically of the same composition except that levels of expoxidized soybean oil were varied, as well as amounts of dioctyl adipate (DOA) and butylated hydroxy toluene (BHT). The only sample within the scope of the present invention was sample 7. The samples were prepared by melting the polyvinyl chloride composition, forming the composition into pressed plaques two inches by two inches in width and about 0.040 inches in thickness, and subjecting the plaques to levels of gamma radiation of 2.5 Mrads and 5.0 Mrads respectively.

The following Table I gives the composition for the several samples and results obtained:

Table 1

| Sample | (1) | (2) | (3) | (4) | (5) | (6) | (7) |
|---|---|---|---|---|---|---|---|
| Polyvinyl Chloride | 100 | → | → | → | → | → | → |
| Proc. aid | 2 | → | → | → | → | → | → |
| Pigments | .145 | → | → | → | → | → | → |
| Calcium/zinc stabilizer | 0.3 | → | → | → | → | → | → |
| Ester lubricant | 1 | → | → | → | → | → | → |
| Co-stabilizer | 0.5 | → | → | → | → | → | → |
| Phosphite | 0.5 | → | → | → | → | → | → |
| MBS impact modifier | 13 | → | → | → | → | → | → |
| Epoxidized soybean oil | 5 | 5 | 5 | 5 | 5 | 10 | 10 |
| Dioctyl adipate | -- | 5 | 10 | -- | 5 | -- | 10 |
| Butylated hydroxy toluene | -- | -- | -- | 1 | 1 | -- | 1 |
| **COLOR** | | | | | | | |
| Original | Clear | Clear | Clear | Clear | Clear | Clear | Clear |
| After 2.5 Mrads gamma radiation | Brown | Brown | Tan | Brown | Light Brown | Brown | Light Brown |
| After 5.0 Mrads gamma radiation | Black | Black | Brown | Black | Brown | Brown | Tan |

The arrows extending from left to right indiciate that the same proportions of listed ingredients were

used as in the control sample 1.

Sample 1 is a conventional polyvinyl chloride composition used for molding hard, rigid parts, containing minimal levels of plasticizer. The result show that the composition turned brown with 2.5 Mrads exposure and black with 5.0 Mrads exposure.

Concerning samples 2 and 3, the use of 5 phr and 10 phr of dioctyl adipate alone gave no improvement (sample 2) or little improvement (sample 3).

Concerning sample 4, the use of BHT alone, also gave no improvement. In sample 5, DOA and BHT were employed at levels of 5 phr and 1 phr, respectively, with only a slight improvement in color drift. Sample 6 with 10 phr of epoxidized soybean oil also showed a slight improvement, about the same as in sample 4. Only in sample 7, with both DOA and BHT, at phr levels of 10 and 1, respectively, was a significant improvement observed. It should be noted that the combination of DOA and BHT in the amounts of 10 phr and 1 phr, respectively, (sample 7) is substantially better than 10 phr of DOA alone (sample 3).

Sample 7 met both toxicity and rigidity requirements of the present invention.

## Example 2

This example illustrates that addition of a tin stabilizer (that employed in this example was a dimethyl tin stabilizer marketed under the trademark "Mark 1984", by Argus Chemical Co.) at 1-5 phr, in combination with an adipate and BHT, offers even further protection against high energy radiation. The level of use of the tin stabilizer was sufficiently low to avoid toxicity. In this example, the DOA plasticizer is present in a higher amount than in Example 1, but it is still at a low enough level that the glass transition temperature is sufficiently high to produce rigid compounds.

The following formulations were prepared, using the procedures of Example 1:

**Table 2**

| Sample | (1) | (2) | (3) | (4) |
|---|---|---|---|---|
| Ingredient | | | | |
| PVC resin | 100 ------------→ | | | |
| Acrylic Polymer processing aid | 3 ------------→ | | | |
| Tin stabilizer | 2 ------------→ | | | |
| Lubricant | 0.2 ------------→ | | | |
| DOA | 15 ------------→ | | | |
| BHT | 1 | -- | -- | -- |
| BHA | -- | 1 | -- | -- |
| Hydroquinone | -- | -- | 1 | -- |
| Propylgallate | -- | -- | -- | 1 |
| Yellowness Index | | | | |
| Before radiation | 4.70 | 2.15 | 2.64 | 22.66 |
| After 5 Mrads radiation | 7.85 | 20.16 | 19.76 | 72.68 |

The above data show that the combination of a tin stabilizer (sample 1) with DOA and BHT produced a product which resists yellowing to a significant degree. A yellowness increase of only about 3.15 was obtained.

The use of compounds structurally similar to BHT, such as BHA, propagallate, and hydroquinone failed to offer the degree of protection afforded by the use of BHT in the combination of additive ingredients.

Sample 1 met toxicity and rigidity criteria of the present invention.

Example 3

In this example, compositions were prepared similar to Sample 1 of Example 1 except that the DOA was replaced in Samples 2, 3, and 4 by a butyrate and phthalates chemically similar to DOA. The following results were obtained:

Table 3

| Sample | |
|---|---|
| 1. DOA | Clear colorless transparent |
| 2. DOB (dioctylbutyrate) | Clear transparent, slight yellowing |
| 3. DIOP (diisoctylphthalate) | Clear transparent, slight yellowing |
| 4. Butyl benzyl Phthalate | Slight hazy, slight yellowing |
| Samples 1 is within the scope of the present invention. | |

In samples 2, 3 and 4, employing a butyrate or phthalate, the yellowness criteria of the present invention was not met.

The test results in the example were obtained by visual examination after exposure to 5 Mrads of gamma radiation or electron beam.

Example 4

This Example provides further comparative data showing the advantage of use of a methyl-tin stabilizer with DOA and BHT in a pigmented composition.

The following formulations were employed:

| Ingredient | Sample 1 | Sample 2 |
|---|---|---|
| PVC resin | 100 | 100 |
| Processing Aid (Acrylic) | 2 | 3 |
| Lubricant | 1.0 | 0.2 |
| Calcium/zinc stabilizer system | 1.3 | -- |
| FDA Approved Methyl-tin stabilizer | -- | 2 |
| Dioctyl adipate (DOA) | -- | 10 |
| Epoxidized Soybean oil costabilizer | 20 | -- |
| Pigment | 0.35 | .125 |
| Impact Modifier | 13 | 8 |
| BHT | 1 | 1 |
| Yellowness index | | |
| (Initial) | 7.8 | 2.95 |
| (2.5 Mrads) | 44 | -- |
| (5.0 Mrads) | 93 | 7.05 |

This example shows the advantage of use of the three-component system (Sample 2) of the present invention for color stability over compositions where the heat stabilizer is a calcium/zinc system (Sample 1). Sample 2 employing 2 phr of dimethyl tin stabilizer had good heat stability as did Sample 1. At the same time, there was substantially less yellowness increase. Both Samples 1 and 2 broadly are within the scope of the present invention. Samples without the combination of DOA and BHT, compared with Sample 1, would have a substantially higher yellowness index.

## Claims

1. A clear, rigid, vinyl polymer part sterilized by exposure to up to about 5 Mrads of gamma radiation characterized as having:

(a) a rigidity as determined by ASTM D638 of at least about 250,000 psi; and

(b) an increase in yellowness index from radiation, as determined by ASTM 1925-70, of less than about 10.

2. The part of claim 1 wherein said polymer is polyvinyl chloride.

3. The part of claim 1 comprising about 2 phr to about 20 phr of an ether-containing adipate or glutarate having a molecular weight less than about 2,000 and about 0.2 phr to about 5 phr of butylated hydroxy toluene.

4. The part of claim 3 further comprising an alkyl tin stabilizer.

5. The part of claim 4 wherein said adipate or glutarate has a molecular weight less than about 500 and the alkyl group of said tin stabilizer is $C_1$-$C_8$.

6. The part of any of claims 3, 4 or 5 wherein said adipate or glutarate is selected from the group consisting of dioctyl adipate (DOA), dibutoxyethoxyethyl adipate (DBEEA), dibutoxyethyl adipate (DBEA), diisobutyl adipate (DIBA), diisooctyl adipate (DIOA), dibutoxyethoxyethyl glutarate (DBEEG) and dibutoxyethyl glutarate (DBEG).

7. A clear, rigid, vinyl polymer part sterilized by exposure to gamma radiation comprising:

(a) about 2 phr to about 20 phr of an ethercontaining adipate or gluterate having a molecular weight less than about 2,000; and

(b) about 0.2 phr to about 5 phr of butylated hydroxy toluene.

8. The part of claim 7 wherein said vinyl polymer is polyvinyl chloride.

9. The part of claim 8 further comprising a stabilizing amount of an alkyl tin stabilizer.

10. The part of claim 8 further comprising an epoxidized soybean oil.

11. The part of claim 10 further comprising a calcium/zinc stabilizer.

12. The part of any of claims 7, 8, 9, 10, or 11 wherein said adipate or glutarate is selected from the group consisting of dioctyl adipate (DOA), dibutoxyethoxyethyl adipate (DBEEA), dibutoxyethyl adipate (DBEA), diisobutyl adipate (DIBA), diisooctyl adipate (DIOA), dibutoxyethoxyethyl glutarate (DBEEG) and dibutoxyethyl glutarate (DBEG).

13. The part of claim 9 wherein said adipate or glutarate has a molecular weight less than about 500 and the alkyl group of said tin stabilizer is $C_1$-$C_8$.

14. The part of claim 9, exposed to up to about 5 Mrads of gamma radiation, which has a rigidity as determined by ASTM D638 of at least about 250,000 psi and an increase in yellowness index from radiation, as determined by ASTM 1925-70, of less than about 10.

15. A method of making a clear, rigid vinyl polymer part which retains clarity when sterilized by exposure to gamma radiation comprising adding to a mix of a vinyl polymer, prior to molding, about 2 phr to about 20 phr of an ether-containing adipate or glutarate having a molecular weight less than about 2,000 and about 0.2 phr to about 5 phr of butylated hydroxy toluene.

16. The method of claim 15 wherein the composition further comprises an alkyl tin stabilizer.

17. The method of claim 16 wherein said adipate or glutarate has a molecular weight less than about 500 and the alkyl group of said tin stabilizer is $C_1$-$C_8$.

18. The method of claim 17 wherein said vinyl polymer is polyvinyl chloride.

19. The method of any of claims 15, 16, 17 or 18 wherein said adipate or glutarate is selected from the group consisting of dioctyl adipate (DOA), dibutoxyethoxyethyl adipate (DBEEA), dibutoxyethyl adipate (DBEA), diisobutyl adipate (DIBA), diisooctyl adipate (DIOA), dibutoxyethoxyethyl glutarate (DBEEG) and dibutoxyethyl glutarate (DBEG).

20. A part prepared by the method of any of claims 15, 16, 17 or 18 which has a rigidity as determined by ASTM D638 of at least about 250,000 psi and an increase in yellowness index from radiation, as determined by ASTM 1925-70, of less than about 10.

European Patent Office

# EUROPEAN SEARCH REPORT

Application Number

EP 89 11 0021

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X,D | SOC. OF PLASTICS ENG., TECH. PAPERS, 1985, no. 31, pages 1068-1071; D.S. HOUSEL: "Factors influencing color drift of gamma sterilized PVC articles" * Sample preparation, plasticizers, antioxidants, tables 2,4 * | 3-19 | C 08 L 27/06<br>C 08 K 5/00<br>A 61 L 2/08 //<br>(C 08 K 5/00<br>C 08 K 5:11<br>C 08 K 5:13 ) |
| X | FR-A-2 078 616 (M&T CHEMICALS INC.) * Example 1; page 3, lines 7-15; page 4, lines 3-40 * | 15-20 | |
| A | M. SZYCHER: "Biocompatible polymers, metals and composites", 18th March 1983, pages 975-999, chapter 43; H. LANDFIELD: "Sterilization of medical devices based on polymer selection and stabilization techniques", 1983 Technomic Publ. Co., Lancaster, PA, US * Page 993, lines 3-11 * | 3-19 | |
| | | | **TECHNICAL FIELDS SEARCHED (Int. Cl.4)** |
| | | | C 08 K<br>C 08 L<br>A 61 L |

The present search report has been drawn up for all claims

| Place of search | Date of completion of the search | Examiner |
| --- | --- | --- |
| THE HAGUE | 18-09-1989 | WILSON A.J.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)